# EUROPÄISCHE PATENTANMELDUNG

(11) **EP 3 693 137 A1**
(43) Veröffentlichungstag der Anmeldung: **12.08.2020**
(21) Anmeldenummer: 19155502.8
(22) Anmeldetag: 05.02.2019
(51) Int. Cl.: B25J 9/16

(54) **VERFAHREN ZUM HERSTELLEN EINES PFADPLANUNGSMODULS UND ZUM BETREIBEN EINES ROBOTERS, PFADPLANUNGSMODUL UND ROBOTER**

(71) Anmelder: Siemens Healthcare GmbH, 91052 Erlangen (DE)
(72) Erfinder: Maschke, Michael, 91475 Lonnerstadt (DE); Pfister, Marcus, 91088 Bubenreuth (DE)

(57) **Zusammenfassung**

Die Erfindung betrifft Verfahren (35) zum Herstellen eines Pfadplanungsmoduls (24) und zum Betreiben eines Roboters (1, 11), das entsprechende Pfadplanungsmodul (24) und den entsprechenden Roboter (1, 11). Es ist vorgesehen, dass das Pfadplanungsmodul (24) eine KI-Komponente (25) aufweist, welche anhand von vorgegebenen Eingangsdaten und einer Trainingszielvorgabe darauf trainiert wird, unsichere Bewegungen des Roboters (11) von sicheren Bewegungen zu unterscheiden und einen jeweiligen Pfad von einer Ausgangspose des Roboters (11) zu einem jeweiligen Ziel zu bestimmen, dem der Roboter (11) unter Ausführung lediglich sicherer Bewegungen folgen kann.

## Beschreibung

Die Erfindung betrifft ein Verfahren zum Herstellen eines Pfadplanungsmoduls für einen Roboter zum Planen einer Bewegung des Roboters. Die Erfindung betrifft weiterhin ein Verfahren zum entsprechenden Betreiben eines Roboters, den entsprechenden Roboter sowie das entsprechende Pfadplanungsmodul.

Roboter finden heutzutage in einer großen Vielfalt technischer Gebiete und Anwendungen Einsatz. Beispielsweise werden Roboter sowohl in der vollautomatischen industriellen Fertigung als auch im medizinischen Umfeld für roboterunterstützte Untersuchungen oder Behandlungen eingesetzt. Dabei können Roboter zum schnellen und effektiven Bewegen von Bauteilen oder Instrumenten und zu deren positionsgenauem Halten leistungsstarke Antriebe und für komplexe Bewegungen ausgelegte bewegliche Teile wie etwa einen mehrgelenkigen oder mehrachsigen Roboterarm aufweisen.

Roboter können vorteilhaft besonders effizient, genau und zuverlässig arbeiten und können eine erhebliche Erleichterung oder Entlastung für menschliches Personal bedeuten. Nicht zuletzt aufgrund der komplexen, gegebenenfalls automatisch gesteuerten oder autonom durchgeführten Bewegungen ist nachteilig jedoch stets Potenzial für Kollisionen des jeweiligen Roboters mit Objekten oder Personen in seiner Umgebung gegeben. Hier können die an sich vorteilhaften schnellen Bewegungen und leistungsstarken Antriebe von Robotern dann ein Beschädigungs- oder Verletzungsrisiko darstellen. Gerade im medizinischen Umfeld werden heutzutage einerseits zunehmend mehr Roboter eingesetzt, andererseits aber besonders hohe Anforderungen hinsichtlich einer Absicherung gegenüber Kollisionen gestellt. Ein Beispiel für multiaxial motorisch bewegliche robotische Einrichtungen für den medizintechnischen Anwendungsbereich ist in der US 8,727,618 B2 beschrieben.

Es gibt bereits verschiedene Ansätze, um Kollisionen von Robotern mit anderen Objekten zu vermeiden. So beschreibt etwa die US 8,660,694 B2 ein Verfahren zum Planen einer Bewegung eines Roboters. Um eine Berechnungszeit für eine kollisionsfreie Bewegungstrajektorien des Roboters zu reduzieren, wird dabei dem Roboter ein Raumpunkt in einem stationären festen Basiskoordinatensystem zugewiesen. Vorgegebene räumliche Positionen dieses Raumpunktes dienen dann als Basis zum Berechnen einer Geometrie des Roboters und zum Umrechnen in entsprechende Konfigurationen in einem Konfigurationsraum des Roboters. Schließlich wird eine Trajektorie entlang vorgegebener räumlicher Positionen berechnet basierend auf einer Überprüfung der Geometrie des Roboters in Bezug auf die Konfigurationen hinsichtlich einer Kollision.

Ein weiterer Ansatz ist aus der US 7,857,512 B2 bekannt. Dort ist ein Kollisionsschutzelement für einen Patiententisch eines medizinischen Röntgengeräts beschrieben, welches zumindest einen Teil eines Patienten mechanisch abschirmen kann. Da das Kollisionsschutzelement aus einem röntgentransparenten Material gebildet ist, wird dabei eine röntgengestützte Abbildung des Patienten nicht beeinträchtigt.

Aufgabe der vorliegenden Erfindung ist es, die Sicherheit und Effizienz beim Einsatz eines beweglichen Roboters zu verbessern. Diese Aufgabe wird erfindungsgemäß durch die Gegenstände der unabhängigen Patentansprüche gelöst. Vorteilhafte Ausgestaltungen und Weiterbildungen der vorliegenden Erfindung sind in den abhängigen Patentansprüchen, in der Beschreibung und in den Figuren angegeben.

Ein erfindungsgemäßes Verfahren dient zum Erzeugen oder Herstellen eines Pfadplanungsmoduls für einen Roboter zum Planen einer automatisch gesteuerten motorischen Bewegung des Roboters. Als Pfad kann dabei beispielsweise eine kontinuierliche Trajektorie oder eine Reihe von nacheinander einzunehmenden Posen verstanden werden. Um die Bewegung auszuführen, kann der Roboter dann diesem Pfad folgen. Das Pfadplanungsmodul kann in Software und/oder in Hardware realisiert werden. Für einen tatsächlichen Betrieb des Pfadplanungsmoduls und des Roboters kann das Pfadplanungsmodul als Teil des Roboters in diesen integriert sein oder über eine Datenverbindung mit dem Roboter verbunden werden. Das Pfadplanungsmodul kann bevorzugt für einen medizinischen oder medizintechnischen Roboter vorgesehen sein, beispielsweise für einen Röntgenroboter, welcher eine Röntgenstrahlquelle und einen gegenüberliegenden Röntgendetektor im Raum positionieren kann, oder für einen Leichtbauroboter, welcher beispielsweise zum Führen oder Halten medizinischer Instrumente bei einer roboterunterstützten Untersuchung oder Intervention eingesetzt werden kann. Die vorliegende Erfindung ist jedoch nicht auf diese medizinischen oder medizintechnischen Anwendungen beschränkt, sondern kann ebenso in anderen industriellen oder technischen Bereichen eingesetzt werden.

Das erfindungsgemäße Verfahren zum Herstellen des Pfadplanungsmoduls umfasst mehrere Verfahrensschritte, die insbesondere ganz oder teilweise computerimplementiert sein können. In einem solchen Verfahrensschritt wird eine trainierbaren künstliche Intelligenz-Komponente, hier kurz bezeichnet als KI-Komponente, bereitgestellt. Die künstliche Intelligenz (englisch: Artificial Intelligence, AI) umfasst mehrere Teilgebiete, sodass dementsprechend unterschiedliche Arten von KI-Komponenten eingesetzt werden können, beispielsweise eine Maschinenlerneinrichtung oder ein Maschinenlernalgorithmus oder ein neuronales Netz. Besonders bevorzugt umfasst die KI-Komponente ein tiefes neuronales Netz, welches eine oder mehrere verborgene Schichten aufweist und fachüblich auch als DLNN (Deep Learning Neural Network) bezeichnet wird. Die bereitgestellte trainierbaren KI-Komponente kann dabei zunächst ungelernt oder untrainiert, beispielsweise mit zufälligen Gewichten oder Parametern initialisiert, sein. Es gibt bereits eine Anzahl von Architekturen für geeignete neuronale Netze und Maschinenlernalgorithmen oder Maschinenlernprogramme, welche als die KI-Komponente für die vorliegende Erfindung verwendet werden können.

Ein weiterer Verfahrensschritt des erfindungsgemäßen Verfahrens ist ein Trainieren der KI-Komponente für das Planen der Bewegung. Dazu werden der KI-Komponente vorgegebene Eingangsdaten und eine Trainingszielvorgabe für eine Bewertung der Eingangsdaten zugeführt. Die Eingangsdaten können auch als Trainingsdaten bezeichnet werden oder Trainingsdaten umfassen. Konkret umfassen die Eingangsdaten erfindungsgemäß Bewegungsdaten, welche Beispielbewegungen des Roboters beschreiben, zugehörige Kollisionsdaten, welche bevorstehende und/oder erfolgte Kollisionen bei den Beispielbewegungen angeben, und zugehörige Zielortvorgaben für die Beispielbewegungen.

Die Beispielbewegungen können in Form von Pfaden, Teilbewegungen, Zustands- oder Posendaten, entsprechenden Sensordaten und/oder dergleichen mehr vorliegen. Die Beispielbewegungen können synthetische, also künstlich oder computergestützt generierte, Daten sein und/oder Daten von in der Vergangenheit tatsächlich erfolgten, beispielsweise von einem ähnlichen Roboter ausgeführten, Bewegungen beschreiben.

Die Kollisionsdaten können beispielsweise in Form von Sensordaten einer Kollisionssensorik vorliegen. Dies können beispielsweise von Kraftsensoren, Berührungssensoren, induktiven Sensoren, kapazitiven Sensoren oder dergleichen erzeugte Sensordaten sein, welche tatsächliche Kollisionen oder eine Kollisionsgefahr angebende Annäherungen des Roboters an Fremdobjekte angeben oder beschreiben.

Zusätzlich oder alternativ können die Kollisionsdaten beispielsweise Annotierungen oder Label der Bewegungsdaten sein oder umfassen. Derartige Annotierungen können dabei, beispielsweise gestützt auf die genannten Sensordaten, automatisch erzeugt und/oder manuell mit den Bewegungsdaten verknüpft werden.

Die Zielortvorgaben geben für jede der Beispielbewegungen ein durch den Roboter dabei zu erreichendes oder anzusteuerndes Ziel an, welches beispielsweise von jeweiligem Bedienpersonal vorgegeben sein oder in Abhängigkeit von einer dem jeweiligen Roboter oder einer jeweiligen Robotersteuerung gestellten Aufgabe automatisch bestimmt worden sein kann. Insbesondere können die Bewegungsdaten, insbesondere die Beispielbewegungen, eine jeweilige Ausgangspose des Roboters zu Beginn der jeweiligen Beispielbewegung angeben, sodass sich der Roboter während der jeweiligen Beispielbewegung also von dieser Ausgangspose bis zu dem jeweiligen vorgegebenen Ziel oder Zielort oder auf dem Weg dahin bis zu einer jeweiligen Kollision oder einer Zwischenposition, an welcher eine Kollisionsgefahr erkannt wurde, bewegt beziehungsweise bewegt hat oder bewegen soll.

Die Trainingszielvorgabe umfasst, unsichere Bewegungen, die zu einer Kollision des Roboters mit einem Fremdobjekt führen oder führen können, von sicheren, kollisionsfreien Bewegungen des Roboters zu unterscheiden und einen jeweiligen Pfad von einer jeweiligen Ausgangspose des Roboters zu dem jeweiligen vorgegebenen Ziel oder Zielort zu finden, dem der Roboter unter Ausführung lediglich sicherer Bewegungen folgen kann. Letzteres kann bedeuten, dass die KI-Komponenten selbst den Pfad generiert oder bereitgestellte Pfade oder zugehörige Bewegungen hinsichtlich ihrer Sicherheit oder Kollisionsfreiheit bewertet. Dabei können von einem herkömmlichen Pfaderzeugungsalgorithmus dann solange unterschiedliche Pfade erzeugt und der KI-Komponente zur Bewertung zugeführt werden, bis ein sicherer Pfad gefunden ist.

Die Trainingszielvorgabe kann also als Referenz für die KI-Komponente oder deren Training dienen. Anhand der Trainingszielvorgabe kann also, insbesondere automatisch, überprüft werden, ob eine während des Trainierens, also während eines Lernprozesses der KI-Komponente, von dieser vorgeschlagene Bewegung oder Pfadplanung anforderungsgerecht ist, also durch den Roboter kollisionsfrei angewendet oder befolgt werden kann. Zum Trainieren der KI-Komponente können grundsätzlich aus dem technischen Gebiet der künstlichen Intelligenz oder des maschinellen Lernens bekannte Trainings- oder Lernmethoden angewendet werden, wie beispielsweise überwachte oder und überwachte Rückpropagation oder dergleichen.

Weitere Details zum Trainieren von KI-Komponenten beziehungsweisen neuronalen Netzen allgemein können beispielsweise entnommen werden aus der US 2017 / 0 098 172 A1, aus der Veröffentlichung "A Fast Learning Algorithm for Deep Belief Nets" von G. E. Hinton et al., Neural Computation 18, 1527-1554 (2006) und aus der Veröffentlichung "The Perceptron: A Probabilistic Model For Information Storage And Organization In The Brain" von F. Rosenblatt, Psychological Review Vol. 65, No. 6, 386-408, 1958.

In einem weiteren Verfahrensschritt des erfindungsgemäßen Verfahrens wird die trainierte KI-Komponente mit zur Kommunikation mit dem Roboter eingerichteten Eingangs- und Ausgangsschnittstellen als das Pfadplanungsmodul bereitgestellt. Die Schnittstellen können also als Interface zwischen dem Pfadplanungsmodul und dem Roboter oder anderen Komponenten des Roboters, beispielsweise einem Steuergerät oder einem Antrieb des Roboters, dienen. Dabei können die Schnittstellen insbesondere für eine bidirektionale Kommunikation eingerichtet sein, wobei das Pfadplanungsmodul beziehungsweise die KI-Komponente über die Schnittstellen als Eingangsdaten beispielsweise einen aktuellen Zustand oder eine aktuelle Pose, also aktuelle Positions- oder Geometriedaten, des Roboters, Sensordaten von einer Sensorik des Roboters und/oder dergleichen mehr empfangen und als Ausgangsdaten den bestimmten oder geplanten Pfad ausgeben kann. Ebenso kann das Pfadplanungsmodul weitere Eingangsdaten, wie beispielsweise eine jeweilige Zielortvorgabe, Umgebungsdaten und/oder Beschränkungen oder Randbedingungen, welche bei dem Bewegen des Roboters einzuhalten sind und/oder dergleichen mehr empfangen und bei dem Planen des Pfades berücksichtigen. Die Schnittstellen des Pfadplanungsmoduls können dabei ebenso wie das Pfadplanungsmodul oder die KI-Komponente insgesamt in Software und/oder in Hardware realisiert sein.

Das Bereitstellen des Pfadplanungsmoduls kann dementsprechend beispielsweise ein Software-Packaging, ein Aufspielen der Software-KI-Komponente auf ein entsprechendes Hardware-Speichermodul, ein Erzeugen einer dedizierten HardwareSchaltung, welche eine Funktion der trainierten KI-Komponente in Hardware umsetzt, und/oder dergleichen mehr bedeuten oder umfassen. Das Pfadplanungsmodul kann ebenso weitere Bauteile oder Komponenten, wie beispielsweise einen computerlesbaren Speicher, weitere Schnittstellen, beispielsweise für Benutzereingaben, Systemeingaben oder Testzwecke oder dergleichen, und/oder dergleichen mehr aufweisen.

Die hier vorgeschlagene Kombination aus Techniken der Bereiche Robotik und Roboter-Pfadplanung sowie künstliche Intelligenz stellt einen neuen Ansatz dar, welche erst seit relativ kurzer Zeit aufgrund entsprechender Fortschritte im Bereich leistungsfähiger Berechnungshardware und Datenverarbeitung überhaupt realisierbar ist und daher bisher nicht in Betracht gezogen wurde. Gegenüber bisher verwendeten Methoden - beispielsweise einer mechanischen Abschirmung zur Kollisionsvermeidung oder einer starren Programmierung genau definierter und vorgegebener Trajektorien für den Roboter - mag die vorliegend vorgeschlagene Lösung zunächst weniger verlässlich erscheinen. Es hat sich jedoch gezeigt, dass die vorliegende Erfindung tatsächlich eine höhere Sicherheit und Zuverlässigkeit zur Vermeidung von Kollisionen von bewegten Robotern oder Roboterkomponenten und gleichzeitig eine verbesserte Flexibilität im Verhalten des Roboters bieten kann. So können bei einem praktischen Einsatz von Robotern immer wieder unvorhergesehene Situationen auftreten, welche mittels einer konventionellen starren Programmierung nicht abgedeckt werden können. Ebenso können durch Systeme zur sensorgestützten Kollisionsvermeidung, welche bei einer Annäherung des Roboters an ein Fremdobjekt den Roboter anhalten, zum einen kollisionsgefährliche Situationen nicht immer zuverlässig vermieden werden und zum anderen ein flüssiger unterbrechungsfreier Arbeitsablauf nicht immer sichergestellt oder erreicht werden. Durch das inhärente Abstraktionsvermögen von Einrichtungen der künstlichen Intelligenz, welche gelerntes Wissen oder gelernte Verhaltensweisen auch auf während des Trainings nicht aufgetretene Situationen anwenden, übertragen oder verallgemeinern können, bietet die vorliegende Erfindung in einer größeren Vielzahl von Situationen die Möglichkeit, den Roboter flexibel an einen jeweiligen Zielort zu steuern und ermöglicht so eine schnellere und effizientere Durchführung robotergestützter Vorgänge. Eine durch die vorliegende Erfindung somit ermöglichte optimierte Robotersteuerung oder Roboterbewegung kann bei einem Einsatz im medizinischen Umfeld beispielsweise Untersuchungs- oder Therapiezeiten verkürzen und so auch zu einer reduzierten Belastung eines jeweiligen Patienten beitragen.

Das erfindungsgemäße Verfahren beziehungsweise dessen Verfahrensschritte können ganz oder teilweise computerimplementiert sein. Die Schritte des erfindungsgemäßen Verfahrens und dessen vorteilhafter Varianten können insbesondere mittels einer Prozessoreinrichtung, beispielsweise einem Mikroprozessor, Mikrochip oder Mikrocontroller, einer Schnittstelle und/oder einer Speichereinheit einer Datenverarbeitungseinrichtung ausgeführt werden. Die Speichereinheit kann dabei beispielsweise ein flüchtiger, also Daten nicht dauerhaft speichernder, Arbeitsspeicher wie Random Access Memory (RAM), ein nicht-flüchtiger, also Daten dauerhaft speichernder Massenspeicher wie eine Festplatte, ein USB-Stick, eine SD-Karte oder eine Solid State Disk (SSD) oder dergleichen sein oder umfassen.

In vorteilhafter Ausgestaltung der vorliegenden Erfindung ist es vorgesehen, dass die Bewegungsdaten von dem Roboter während der Beispielbewegung eingenommene Posen aufgelöst nach einzeln beweglichen Segmenten oder Komponenten des Roboters angeben. Mit anderen Worten kann also beispielsweise nicht nur eine Position oder Bewegung beispielsweise eines Schwerpunkts oder Mittelpunkts oder eines Endeffektors des Roboters oder eines Roboterarms angegeben werden, sondern für einzelne Segmente oder Komponenten des Roboters können deren individuelle Bewegungen angegeben sein. Dies ist besonders vorteilhaft, da beispielsweise bei einer Schwenk- oder Knickbewegung, bei der unterschiedliche Segmente des Roboters relativ zueinander bewegt werden, wenigstens eines der Segmente, gegebenenfalls entgegen einer Gesamtbewegungsrichtung des Roboters, ausschwenken kann und mit einem solchen Ausschwenken ein besonderes Kollisionsrisiko verbunden sein kann. Beispielsweise kann sich ein Roboterarm aus mehreren Segmenten insgesamt von einem Fremdobjekt wegbewegen, dabei jedoch beispielsweise ein von einem Endeffektor abgewandtes hinteres Ende eines abknickenden Segments des Roboterarms sich auf das Fremdobjekt oder ein anderes Objekt, beispielsweise Personal, zu bewegen, was für sich in einer Umgebung oder einem Arbeitsbereich des Roboters aufhaltende Personen oftmals nicht ohne Weiteres vorhersehbar ist. Ebenso kann der Roboter beispielsweise mit austauschbaren Komponenten ausgestattet sein, welche unterschiedliche Ausdehnungen, Bewegungsfreiheitsgrade oder Verhalten haben können. Insgesamt ermöglicht die segment- oder komponentenspezifische aufgelöste Angabe der Positionen und/oder Posen des Roboters vorteilhaft eine besonders zuverlässige Kollisionsvermeidung. Insbesondere können die einzelnen Posen jeweilige Positionen von ausgezeichneten, am weitesten außen liegenden Punkten, beispielsweise Endpunkten oder hervorstehenden Teilen, angeben. Dies ist insbesondere im Gegensatz zu Positionsangaben zu sehen, welche oftmals vereinfachend nur eine Gelenksposition oder eine Schwerpunktposition oder eine Richtung einer Längsachse eines Segments zwischen zwei Gelenken angeben, wobei herkömmlicherweise gegebenenfalls unberücksichtigt bleibt, dass das tatsächliche reale Segment über diese Angaben hinausragen kann.

In weiterer vorteilhafter Ausgestaltung der vorliegenden Erfindung umfasst das Trainieren der KI-Komponente ein Vortrainieren und ein späteres Feinabstimmen oder Feintuning anhand von ortsspezifischen Eingangsdaten, die spezifisch sind für einen individuellen Einsatzort des jeweiligen Roboters. Mit anderen Worten kann das Vortrainieren beispielsweise herstellerseitig an einem Herstellungs- oder Produktionsort des Pfadplanungsmoduls oder des Roboters durchgeführt werden. Das Feinabstimmen kann dann beispielsweise nach Auslieferung und Installation des Pfadplanungsmoduls und des Roboters an einem letztendlichen Einsatz- oder Betriebsort des Roboters erfolgen. Die ortsspezifischen Eingangsdaten können dabei beispielsweise jeweilige räumliche Bedingungen oder Gegebenheiten an dem jeweiligen individuellen Einsatzort angeben oder berücksichtigen. So kann die KI-Komponente vorteilhaft individuell an den jeweiligen Einsatzort angepasst werden. Dadurch kann beispielsweise berücksichtigt werden, dass eine bestimmte Bewegung oder ein bestimmter Pfad an einem ersten Einsatzort sicher sein kann, also Kollision frei durchgeführt oder befolgt werden kann, an einem anderen Einsatzort hingegen unsicher sein, also zu einer Kollision oder Kollisionsgefahr führen kann.

Da das Feinabstimmen erfahrungsgemäß wesentlich schneller und mit weniger Aufwand durchgeführt werden kann, wenn die KI-Komponente bereits vortrainiert ist, kann die Sicherheit und Effizienz beim Einsatz des jeweiligen Roboters durch die vorliegend vorgeschlagene Ausgestaltung der vorliegenden Erfindung weiter verbessert werden. Dies ist damit begründet, dass der Roboter an seinem letztendlichen Einsatzort durch die durch das bereits erfolgte Vortrainieren verkürzte für das Feinabstimmen benötigte Zeit effizienter einsatzbereit gemacht, also zum Einsatz kommen kann. Zum anderen muss beispielsweise für das zeitintensive Vortrainieren ein Arbeitsbetrieb an dem jeweiligen Einsatzort nicht unterbrochen werden. Würde hingegen das gesamte Training der KI-Komponente an dem individuellen Einsatzort des Roboters durchgeführt werden, so könnte dies in der Praxis nachteilig dazu führen, dass das Training verkürzt wird, um eine Produktionspause möglichst gering zu halten, worunter wiederum die Sicherheit beim späteren Einsatz oder Betrieb des Roboters leiden könnte.

In vorteilhafter Weiterbildung der vorliegenden Erfindung ist es vorgesehen, dass die ortsspezifischen Eingangsdaten räumliche Bedingungen und für den individuellen Roboter an dessen individuellem Einsatzort vorgesehene Arbeitsabläufe beschreiben. Mit anderen Worten werden für das Feinabstimmen also nicht nur räumliche oder geometrische Bedingungen oder Einschränkungen an dem jeweiligen Einsatzort berücksichtigt, sondern auch eine Art und Weise, wie der Roboter dort voraussichtlich eingesetzt oder verwendet werden wird. Die ortsspezifischen Eingangsdaten können bezüglich der Arbeitsabläufe beispielsweise typischerweise oder voraussichtlich anwesendes Personal, dessen bevorzugte Positionen und Bewegungen, weitere genutzte Ausrüstung, insbesondere weitere an dem Einsatzort vorhandene oder eingesetzte mobile Geräte, deren Abmessung, Positionen und Bewegungen, für einen Arbeitsablauf benötigte Freiräume und/oder dergleichen mehr umfassen oder angeben.

Beispielsweise können verschiedene Teams im medizinischen Anwendungsfeld, beispielsweise verschiedene OP-Mannschaften, unterschiedliche individuelle Arbeitsabläufe haben oder bevorzugen, auf die sie eingespielt sind. Zudem können beispielsweise zwei augenscheinlich identische Roboter beispielsweise jeweils ausschließlich für unterschiedliche Therapien oder Untersuchungsmethoden eingesetzt werden, woraus sich unterschiedliche Bewegungsabläufe sowie unterschiedliche Arbeitsabläufe einschließlich unterschiedlicher Anwesenheiten, Einsatzzeiten, Kombinationen und Bewegungen von Personal und weiteren Geräten ergeben können. Derartige ortsspezifische Eingangsdaten können dementsprechend eine individuelle Feinabstimmung des Roboters ermöglichen, durch welche die Sicherheit und Effizienz beim Einsatz des Roboters an seinem individuellen Einsatzort weiter verbessert werden kann. Demgegenüber sind derartige Optimierungen einer Robotersteuerung beispielsweise auf von jeweiligem Personal an einem jeweiligen Einsatzort bevorzugte Arbeitsabläufe bei herkömmlichen automatischen Robotersteuerungen gemäß fest vorgegebener Trajektorien nicht praktikabel realisierbar.

In weiterer vorteilhafter Ausgestaltung der vorliegenden Erfindung ist es vorgesehen, dass das Trainieren der KI-Komponente während eines produktiven Einsatzes des Roboters anhand von Bewegungsdaten dieses Roboters fortgesetzt wird. Mit anderen Worten ist es also vorgesehen, dass mit Abschluss des Trainings oder einer Trainingsphase im Rahmen des Herstellungsprozesses des Planungsmoduls vor dessen Inbetriebnahme durch einen Endkunden die Gewichte oder Parameter der KI-Komponente nicht finalisiert, also fest eingestellt werden und damit also weiterhin dynamisch anpassbar bleiben. Damit kann die KI-Komponente auch nach Beginn des produktiven Einsatzes, am Beispiel eines mittels des Pfadplanungsmoduls betriebenen medizintechnischen Roboters also etwa nach Aufnahme eines regulären Patientenbetriebes, weitere sichere und/oder mit Kollisionen oder Kollisionsgefahren verbundene Bewegungsvorgänge des Roboters lernen. Daraufhin kann die KI-Komponente durch einen automatischen, insbesondere nichtüberwachten, Lern- oder Optimierungsprozess die Pfadplanung und damit eine letztendliche Steuerung von Bewegungen oder Bewegungsabläufen des Roboters weiter verbessern und an einen individuellen Einsatzort und Einsatzzwecken des jeweiligen Roboters anpassen. Dies ermöglicht vorteilhaft eine verbesserte Flexibilität des Pfadplanungsmoduls und kann somit eine verbesserte Sicherheit und Effizienz beim Einsatz des Roboters in unterschiedlichsten Anwendungsszenarien ermöglichen.

In vorteilhafter Weiterbildung der vorliegenden Erfindung werden für das fortgesetzte Training der KI-Komponente Bewegungsdaten, welche tatsächlich von dem Roboter ausgeführte Bewegungen beschreiben, die zu einer Kollision oder einer Kollisionsgefahr geführt haben, dem Pfadplanungsmodul automatisch als als unsicher gekennzeichnete Trainingsdaten zugeführt. Da bei einem Betrieb des Roboters die Kollisionsvermeidung oberste Priorität hat, kann durch die Fokussierung auf unsichere oder gefährliche Bewegungen, die zu vermeiden sind, besonders effizient und zuverlässig eine Optimierung der Pfadplanung hinsichtlich einer Kollisionsvermeidung erreicht werden. Die tatsächlich ausgeführten Bewegungen können beispielsweise anhand von Sensor- oder Zustandsdaten und/oder Steuersignalen einer Robotersteuerung oder eines Steuergeräts des Roboters beschrieben oder nachvollzogen und in die Bewegungsdaten umgewandelt werden.

Das Kennzeichnen als unsichere Bewegung kann beispielsweise automatisch anhand von Sensordaten einer Kollisionssensorik erfolgen. Diese Kollisionssensorik kann bevorzugt Teil des Roboters sein. Ebenso kann die Kollisionssensorik aber eine externe Sensorik sein oder umfassen, beispielsweise eine Sensorik eines in einem Arbeitsbereich oder einer Umgebung des Roboters befindlichen weiteren Gerätes oder eine - etwa kamera-, radar- oder schallgestützte - Raumüberwachungssensorik eines Raumes, in dem der Roboter arbeitet. Ebenso können von dem Roboter ausgeführte Bewegungen beispielsweise durch ein Personal manuell als unsicher oder zu vermeidende Bewegungen gekennzeichnet werden. Dazu kann der Roboter oder das Pfadplanungsmodul beispielsweise eine entsprechende Schnittstelle oder ein entsprechendes Bedienelement zum Empfangen von entsprechenden Benutzereingaben aufweisen. Der Roboter beziehungsweise das Pfadplanungsmodul kann dann dazu eingerichtet sein, eine jeweils aktuell oder zuletzt ausgeführte Bewegung des Roboters bei Empfang einer entsprechenden Benutzereingabe automatisch als unsicher zu kennzeichnen und als entsprechend annotierte Trainingsdaten der KI-Komponente zuzuführen. Dies stellt vorteilhaft eine besonders einfach zu realisierende und gleichzeitig besonders effektive Möglichkeit dar, eine Verhaltensweise oder Funktionsweise des Pfadplanungsmoduls an individuelle Gegebenheiten, Bedürfnisse oder Situationen flexibel anzupassen. Dies kann zu einer weiteren Optimierung des Pfadplanungsmoduls für einen jeweiligen individuellen tatsächlichen Einsatzort und Einsatzzweck des Roboters beitragen und damit die Sicherheit und Effizienz beim Einsatz des Roboters weiter verbessern.

In weiterer vorteilhafter Ausgestaltung der vorliegenden Erfindung wird für das Trainieren der Roboter in einem handgeführten, gegebenenfalls durch einen Antrieb des Roboters kraftunterstützten, Betrieb entlang wenigstens einer kollisionsfreien Trajektorie geführt. Diese kollisionsfreie Trajektorie wird dabei aufgezeichnet und, insbesondere automatisch, als sicher gekennzeichnet und dann der KI-Komponente als entsprechend annotierte Eingangsdaten oder Trainingsdaten zugeführt. Mit anderen Worten kann auf diese Weise ein jeweiliges Personal also gemäß individuellen Anforderungen, Bedürfnissen oder Vorlieben als sicher definierte Trajektorien auf besonders einfache Art und Weise vorgeben oder definieren. Somit kann das Pfadplanungsmodul vorteilhaft auch von fachfremdem, also nicht mit einem Programmieren oder Optimieren von KI-Komponenten vertrautem, Personal trainiert und an individuelle Anforderungen oder Situationen angepasst werden.

Besonders bevorzugt können dabei ebenso durch entsprechendes Bewegen, beispielsweise durch Hin- und Herbewegen, des Roboters oder von Teilen des Roboters sichere Raumbereiche definiert werden, in denen sich der Roboter beliebig bewegen darf. Dies ermöglicht vorteilhaft eine flexible und damit besonders effiziente Pfadplanung und entsprechend Bewegung des Roboters, da beispielsweise ein vorgegebener Zielort gegebenenfalls mit unterschiedlichen Posen oder Trajektorien des Roboters erreicht werden kann. Das Trainieren anhand der handgeführten Trajektorien kann besonders vorteilhaft zur Feinabstimmung, also zum Feintuning, des Pfadplanungsmoduls beziehungsweise der KI-Komponente eingesetzt werden auch wenn sich der Roboter und das Pfadplanungsmodul bereits in einem produktiven Einsatz befinden. So kann beispielsweise die Pfadplanung vor einer jeweiligen Untersuchung oder Behandlung eines Patienten, welche sich beispielsweise von einem üblichen Routineeinsatz des Roboters unterscheiden kann, eingesetzt werden. Damit kann besonders einfach und schnell die Pfadplanung an die jeweilige Situation, also einen jeweils bevorstehenden Einsatz angepasst und damit die Sicherheit beim Betrieb des Roboters stets besonders einfach und zuverlässig sichergestellt werden.

Ein weiterer Aspekt der vorliegenden Erfindung ist ein Verfahren zum Betreiben eines motorisch bewegbaren Roboters. Bei diesem Verfahren werden einem erfindungsgemäß hergestellten Pfadplanungsmodul eine aktuelle Ausgangspose des Roboters und ein Bewegungsziel, welches durch die Bewegung erreicht werden soll, vorgegeben. Das Bewegungsziel kann dabei als Zielort oder Zielpose für den Roboter oder ein von dem Roboter geführtes oder gehaltenes Werkzeug oder Instrument ebenso definiert oder vorgegeben werden wie beispielsweise durch eine durchzuführende Untersuchungs- oder Behandlungsmaßnahme. Handelt es sich bei dem Roboter beispielswese um einen Röntgenroboter, so kann als Ziel dann etwa vorgegeben werden, dass ein bestimmter Teilbereich eines jeweiligen Patienten geröntgt werden soll. Das Pfadplanungsmodul kann daraufhin eine dafür notwendige Pose des Roboters und einen zum Erreichen dieser Pose geeigneten Pfad selbsttätig bestimmen. Dazu kann das Pfadplanungsmodul beziehungsweise die KI-Komponente beispielsweise mit entsprechenden Angaben trainiert werden oder trainiert worden sein. Ebenso kann dazu beispielsweise eine vorgegebene Zuordnungstabelle hinterlegt sein, in welcher zu einem derartigen, aufgabenhaft definierten Ziel eine zugehörige Pose für den Roboter definiert ist, welche dann als Zielpose von dem Pfadplanungsmodul zum Bestimmen des Pfades verwendet werden kann.

Als weiterer Verfahrensschritt des erfindungsgemäßen Verfahrens zum Betreiben des Roboters wird durch das Pfadplanungsmodul automatisch ein Pfad bestimmt, entlang welchem der Roboter von der Ausgangspose zum Erreichen des Bewegungsziels durch kollisionsfreie Bewegungen folgen kann. Sowohl hier als auch im Zusammenhang mit den übrigen Aspekten der vorliegenden Erfindung kann dem Pfadplanungsmodul als Parametersatz eine Geometrie und ein möglicher Bewegungsumfangs des Roboters zugeführt oder bereitgestellt werden. Dies stellt vorteilhaft eine besonders einfache Möglichkeit dar, das Pfadplanungsmodul an unterschiedliche Roboter anzupassen, da hierfür dann beispielsweise lediglich dieser Parametersatz ausgetauscht werden muss.

In einem weiteren Verfahrensschritt wird der bestimmte Pfad einem Steuergerät des Roboters bereitgestellt und durch das Steuergerät automatisch in Steueranweisungen für einen Antrieb des Roboters umgesetzt. In einem weiteren Verfahrensschritt wird der Antrieb des Roboters dann automatisch gemäß den Steueranweisungen angesteuert, um den Roboter entlang des bestimmten Pfades zu bewegen.

Als zusätzliche Eingangsdaten oder als zusätzlicher Input können dem Pfadplanungsmodul dabei wie beschrieben beispielsweise aktuelle Gegebenheiten, Bedingungen, Einschränkungen oder Situationsdaten zugeführt werden. Diese können beispielsweise aktuelle Positionen, Abmessungen und Bewegungen von im Arbeitsbereich des Roboters befindlichem Gerät und/oder Personal, Angaben zu geplanten Arbeitsabläufen und/oder dergleichen mehr sein.

Das erfindungsgemäße Verfahren zum Betreiben des motorisch bewegbaren Roboters umfasst also ein Betreiben des Roboters mittels oder unter Verwendung eines erfindungsgemäß hergestellten Pfadplanungsmoduls. Dementsprechend können die im Zusammenhang mit dem erfindungsgemäßen Verfahren zum Herstellen des Pfadplanungsmoduls genannten Maßnahmen oder Abläufe ebenso als - gegebenenfalls optionale - weitere Verfahrensschritte in das erfindungsgemäße Verfahren zum Betreiben des motorisch bewegbaren Roboters integriert oder aufgenommen werden.

Ein weiterer Aspekt der vorliegenden Erfindung ist ein Pfadplanungsmodul für einen motorisch bewegbaren Roboter zum Planen einer automatisch gesteuerten motorischen Bewegung des Roboters. Das erfindungsgemäße Pfadplanungsmodul ist dabei gemäß einem erfindungsgemäßen Verfahren zum Herstellen des Pfadplanungsmoduls hergestellt und/oder zum Betreiben, also für einen Betrieb gemäß einem erfindungsgemäßen Verfahren zum Betreiben eines motorisch bewegbaren Roboters, welcher beispielsweise das erfindungsgemäße Planungsmodul enthalten oder umfassen kann, eingerichtet. Dementsprechend kann das erfindungsgemäße Pfadplanungsmodul also insbesondere die trainierte KI-Komponente aufweisen. Das erfindungsgemäße Pfadplanungsmodul kann also insbesondere das im Zusammenhang mit den übrigen Aspekten der vorliegenden Erfindung genannte Pfadplanungsmodul sein. Dementsprechend kann das erfindungsgemäße Pfadplanungsmodul die im Zusammenhang mit den übrigen Aspekten der vorliegenden Erfindung genannten Eigenschaften und/oder Bauteile oder eine Auswahl davon aufweisen.

Ein weiterer Aspekt der vorliegenden Erfindung ist ein Roboter, welcher einen motorisch bewegbaren Roboterarm, einen Antrieb zum, insbesondere automatischen, Bewegen des Roboterarms und ein mit dem Antrieb verbundenes Steuergerät zum automatischen Ansteuern des Antriebs aufweist. Erfindungsgemäß ist es dabei vorgesehen, dass der erfindungsgemäße Roboter ein erfindungsgemäßes Pfadplanungsmodul aufweist. Dieses Pfadplanungsmodul kann dabei insbesondere Teil des Steuergeräts oder mit dem Steuergerät verbunden sein. Insbesondere kann der erfindungsgemäße Roboter die im Zusammenhang mit dem erfindungsgemäßen Verfahren beschriebene KI-Komponente aufweisen oder über eine Datenverbindung mit einer solchen KI-Komponente verbunden sein. Letzteres kann beispielsweise der Fall sein, wenn die KI-Komponente, also eine entsprechende Software oder ein entsprechender Programmcode, auf einem entfernt von dem Roboter angeordneten Computer, wie etwa einem Server oder einem Rechenzentrum (lokal, on-premise oder über ein wide-area-network (WAN) angebunden), ausgeführt wird. Der erfindungsgemäße Roboter kann zudem weitere Komponenten oder Bauteile aufweisen, wie beispielsweise eine Kollisionssensorik zum Detektieren von Kollisionen oder Annäherungen des Roboters, insbesondere des Roboterarms, an Fremdobjekte, eine Lagesensorik zum Überwachen einer jeweils aktuellen Pose des Roboters, insbesondere des Roboterarms, eine oder mehrere Schnittstellen zum Datenaustausch oder zur Kommunikation, ein Benutzerinterface, ein Bedienelement, und/oder dergleichen mehr.

Der Begriff "Roboterarm" kann im Sinne der vorliegenden Erfindung breit ausgelegt werden und allgemein motorisch bewegbaren Komponenten des Roboters, welche ein Instrument oder ein Werkzeug bilden oder zum Führen oder Halten eines Instruments oder Werkzeugs oder eines Fremdobjekts ausgebildet sind umfassen. So kann als Roboterarm beispielsweise ein herkömmlicher mehrachsiger oder mehrgelenkiger Segmentarm mit beispielsweise wenigstens sechs Freiheitsgraden ebenso wie ein Knickarm oder ein an einem Roboterfuß direkt oder indirekt gehaltener und drehbarer und/oder schwenkbarer C-Arm oder C-Bogen verstanden werden.

Der Antrieb des Roboters kann beispielsweise elektromotorische, hydraulisch oder pneumatische sein.

Das Steuergerät kann bevorzugt einen Datenspeicher und eine Prozessoreinrichtung, beispielsweise einen Mikroprozessor, Mikrochip oder Mikrocontroller, umfassen. Insbesondere sofern das Pfadplanungsmodul nicht als dedizierte Hardwareschaltung vorliegt, kann der Roboter zum Betreiben oder Ausführen des Pfadplanungsmoduls ebenso eine Prozessoreinrichtung aufweisen. Diese kann eine separate Prozessoreinrichtung oder die Prozessoreinrichtung des Steuergeräts sein.

Der erfindungsgemäße Roboter kann insbesondere zum Betreiben gemäß einem erfindungsgemäßen Verfahren zum Betreiben eines motorisch bewegbaren Roboters eingerichtet sein. Der erfindungsgemäße Roboter kann insbesondere der im Zusammenhang mit den übrigen Aspekten der vorliegenden Erfindung genannte Roboter sein. Dementsprechend kann der erfindungsgemäße Roboter die im Zusammenhang mit den übrigen Aspekten der vorliegenden Erfindung genannten Eigenschaften, Bauteile und/oder Komponenten oder eine Auswahl davon aufweisen.

Die bisher und im Folgenden angegebenen Eigenschaften und Weiterbildungen der erfindungsgemäßen Verfahren, des erfindungsgemäßen Pfadplanungsmodul und des erfindungsgemäßen Roboters sind jeweils wechselseitig zwischen diesen Aspekten der vorliegenden Erfindung übertragbar. Es gehören also zu der Erfindung auch solche Weiterbildungen der genannten Aspekte der Erfindung, welche Ausgestaltungen aufweisen, die hier zur Vermeidung unnötiger Redundanz nicht explizit in der jeweiligen Kombination oder nicht für jeden der Aspekte der vorliegenden Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: eine schematische Übersichtsdarstellung zur Veranschaulichung eines robotergestützten System;
- FIG 2: eine schematische Übersichtsdarstellung zur Veranschaulichung eine KI-basierten Pfadplanungsmoduls für einen Roboter; und
- FIG 3: einen beispielhaften schematischen Ablaufplan für ein Verfahren zum Herstellen eines Pfadplanungsmoduls und Betreiben eines Robotersystems.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Elemente jeweils mit den gleichen Bezugszeichen gekennzeichnet.

Bei einem Einsatz von beweglichen Robotern ist eine Kollisionsvermeidung generell eine wichtige Problematik. Gerade im medizinischen Umfeld gibt es bei einigen bekannten Robotersystemen oder für einige Arten von Untersuchungen den Ansatz, mit dem jeweiligen Roboter vor Beginn der eigentlichen Untersuchung eine geplante Trajektorie oder einen geplanten Pfad für den Roboter zunächst in einer Art Probefahrt mit verlangsamter Geschwindigkeit abzufahren und dabei zu beobachten, ob es zu Kollisionen kommt und ob der Roboter eine letztendlich gewünschte Zielposition erreichen kann. Kann der Roboter die Zielposition kollisionsfrei erreichen, wird dann anschließend die eigentliche Untersuchung oder Therapie in Echtzeit, also mit nicht verlangsamter Geschwindigkeit des Roboters ausgeführt. Dies stellt nachteilig einen signifikanten Zusatzaufwand dar und bedeutet eine Zeitverzögerung für die eigentliche Untersuchung. Daneben gibt es Roboter beziehungsweise Robotersteuerungen, welche nach fest vorgegebenen, einmal berechneten und ausgelegten Hardware- und Softwarealgorithmen den Roboter entlang einer vorgegebenen Trajektorie bewegen und diese Bewegung gegebenenfalls bei einer sensorisch detektierten Kollision oder Kollisionsgefahr unterbrechen können. Nachteilig werden dabei aber oftmals nicht sämtliche Oberflächenbereiche oder Komponenten des Roboters berücksichtigt, sodass weiterhin beispielsweise Kollisionen eines hinteren, also von einem Endeffektor abgewandten Zwischengelenks eines Roboterarms auftreten können.

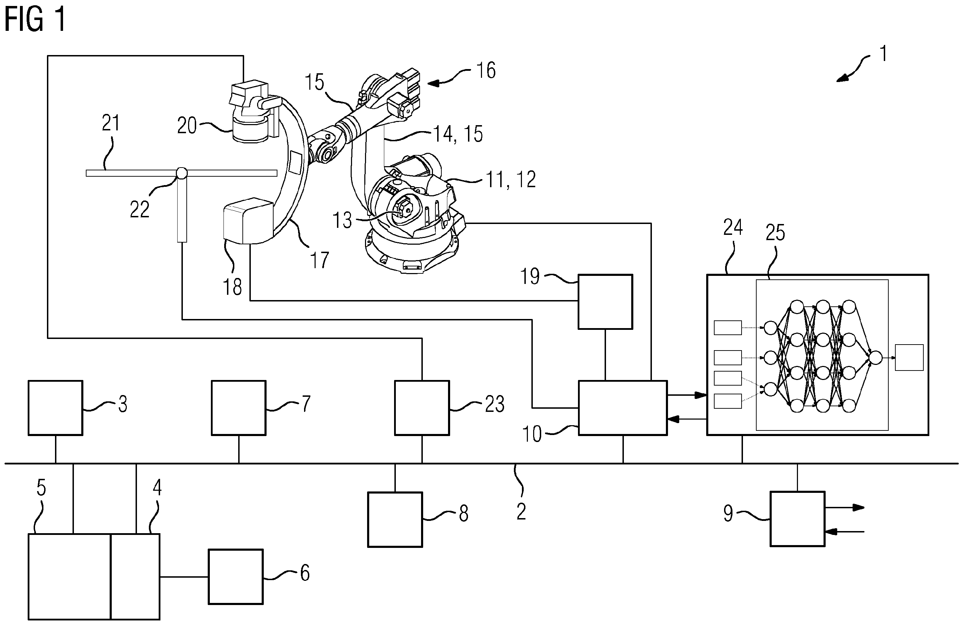

FIG 1 zeigt eine schematische Übersichtsdarstellung zur Veranschaulichung eines Robotersystems 1, durch welches die genannten Probleme gelöst oder vermieden werden können. Das Robotersystem 1 weist hier mehrere jeweils schematisch angedeutete Komponenten auf, die über einen Energie- und Datenbus 2 miteinander verbunden sind. Über eine Energieversorgung 3 werden die einzelnen Komponenten mit Energie versorgt. Über ein Benutzerinterface oder eine Benutzerschnittstelle 4, welche hier mit einer Anzeigeeinrichtung 5 verbunden oder über die Anzeigeeinrichtung 5 zugänglich ist, kann ein jeweiliger Benutzer Arbeitsanweisungen 6 an das Robotersystem 1 übermitteln. Diese Arbeitsanweisungen 6 können beispielsweise eine Auswahl eines durchzuführen Organ- oder Untersuchungsprogramms, eine Zielvorgabe für das Robotersystem 1, benutzer-, anwendungs- und/oder umgebungsspezifische Vorgaben, Kriterien, Einschränkungen oder Parameter und/oder dergleichen sein oder umfassen.

Weiter weist das Robotersystem 1 eine Speichereinrichtung 7 auf, in welcher beispielsweise Betriebsdaten, Anwendungsprogramme, Bilddaten und/oder dergleichen mehr gespeichert sein oder abgelegt werden können. Das Robotersystem 1 weist vorliegend auch eine Prozessor- oder Datenverarbeitungseinrichtung 8 auf, welche insbesondere einen Bildverarbeitungsprozessor oder Bildverarbeitungsalgorithmus umfassen kann. Mittels der Datenverarbeitungseinrichtung 8 können beispielsweise durch das Robotersystem 1 generierte Daten verarbeitet und entsprechende Ergebnisse erzeugt und, beispielsweise an die Anzeigeeinrichtung 5, ausgegeben werden.

Ebenfalls als Teil des Robotersystems 1 ist vorliegend ein DICOM-Interface 9 (Digital Imaging and Communications in Medicine) vorgesehen, über welches das Robotersystem 1 Daten mit einem externen Datensystem, beispielsweise einem Gesundheitsinformationssystem (englisch: Health Information System, HIS) austauschen kann.

Ein ebenfalls an den Datenbus 2 angeschlossenes Steuergerät 10 des Robotersystems 1 ist vorliegend zum Ansteuern eines eigentlichen Roboters 11 eingerichtet. Der Roboter 11 ist hier beispielhaft als Röntgenroboter ausgebildet. Dazu weist der Roboter 11 einen, beispielsweise ortsfest montierten, Roboterfuß 12 auf, in welchem ein Antrieb 13 für einen von dem Roboterfuß 12 ausgehenden beweglichen Roboterarm 14 angeordnet ist. Der Roboterarm 14 weist mehrere Segmente 15 auf, welche über jeweilige Achsen oder Gelenke relativ zueinander beweglich verbunden sind. Beispielhaft ist hier ein Zwischengelenk oder hinteres Gelenk 16 gekennzeichnet. An einem von dem Roboterfuß 12 abgewandten Ende des Roboterarms 14 ist vorliegend ein C-Bogen 17 gehalten oder befestigt. An dem C-Bogen 17 wiederum sind eine Röntgen-Strahlquelle 18, welche durch eine Versorgungseinrichtung 19 des Robotersystems 1 versorgt oder betrieben wird, und ein gegenüberliegender Röntgen-Detektor 20 gehalten. Damit kann ein Untersuchungsobjekt, beispielsweise ein Patient, untersucht oder abgebildet werden. Dazu kann das Untersuchungsobjekt auf einem Patiententisch 21 angeordnet werden, welcher sich in einem Einfluss- oder Arbeitsbereich des Roboters 11 befindet. Vorliegend weist der Patiententisch 21 seinerseits einen Tischantrieb 22 auf, mit welchem der Patiententisch 21, insbesondere relativ zu dem Roboter 11, bewegbar, also räumlich verschiebbar ist. Hierdurch kann eine verbesserte Erreichbarkeit des auf dem Patiententisch 21 angeordneten Untersuchungsobjekts durch den Roboter 11 erreicht werden.

Von dem Detektor 20 erfasste Messdaten können durch eine Detektordatenverarbeitungseinrichtung 23 vorverarbeitet werden. Entsprechende Datenverarbeitungsergebnisse können dann beispielsweise durch die Datenverarbeitungseinrichtung 8 weiterverarbeitet werden, hier etwa zu Röntgenbildern des jeweiligen Untersuchungsobjekts.

Zum Steuern oder Bewegen des Roboters 11 beziehungsweise des Roboterarms 14 und der daran angeordneten Röntgeneinrichtungen ist vorliegend ein Pfadplanungsmodul 24 vorgesehen. Wie durch entsprechende Pfeile hier angedeutet ist, sind das Pfadplanungsmodul 24 und das Steuergerät 10 für einen Datenaustausch, also eine Kommunikation miteinander angeordnet und eingerichtet. Um eine verbesserte Sicherheit und Effizienz beim Einsatz oder Betrieb des Roboters 11 beziehungsweise des Robotersystems 1 zu erreichen, weist das Pfadplanungsmodul 24 vorliegend eine KI-Komponente 25 auf.

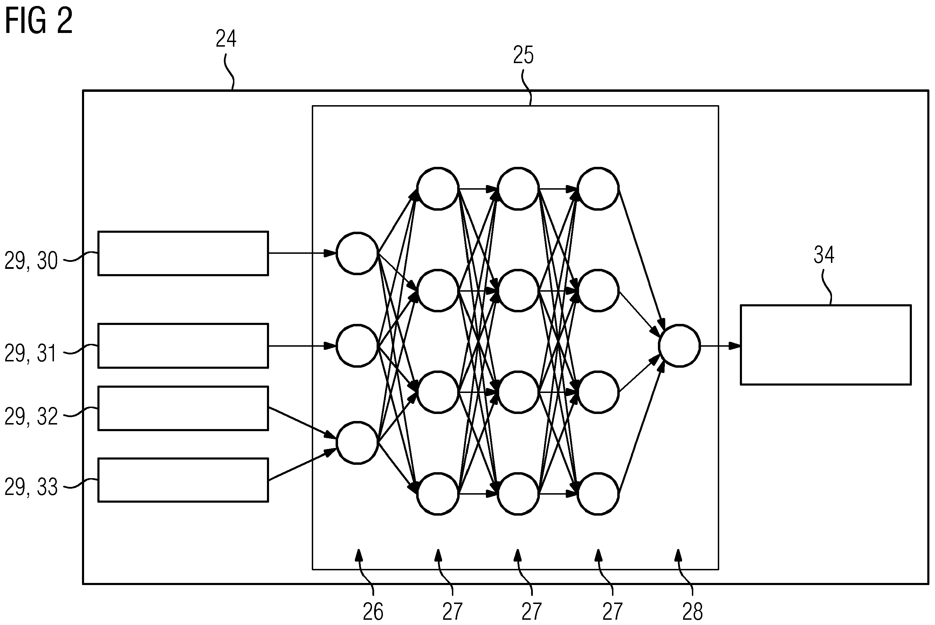

FIG 2 zeigt eine schematische Übersichtsdarstellung zur Veranschaulichung des Pfadplanungsmoduls 24. Dessen KI-Komponente 25 ist hier als tiefes, also mehr als zwei Schichten aufweisendes neuronales Netz, auch bezeichnet als DLNN, ausgebildet. Eine hier dargestellte Struktur der KI-Komponente 25 ist dabei jedoch rein schematisch und beispielhaft zu verstehen, da prinzipiell eine große Vielfalt unterschiedlicher Strukturen, Größen und Dimensionen der KI-Komponente 25 beziehungsweise des DLNN verwendbar sind. Vorliegend weist das DLNN in eine Input- oder Eingangsschicht 26 auf, welcher mehrere verborgene Schichten 27 (englisch: hidden layers) nachgeschaltet sind. Nach einer letzten der verborgenen Schichten 27 ist eine Ausgangsschicht 28 angeordnet. An der Eingangsschicht 26 können dem DLNN beziehungsweise der KI-Komponente 25 hier schematisch angedeutete Eingangsdaten 29 bereitgestellt oder zugeführt werden. Diese Eingangsdaten 29 werden dann durch das DLNN verarbeitet und fließen dabei durch dessen Schichtstruktur von der Eingangsschicht 26 durch die verborgenen Schichten 27 bis hin zu der Ausgangsschicht 28. An der Ausgangsschicht 28 kann das DLNN beziehungsweise die KI-Komponente 25 als Verarbeitungsergebnisse Ausgangsdaten 34, beispielsweise an das Steuergerät 10, bereitstellen.

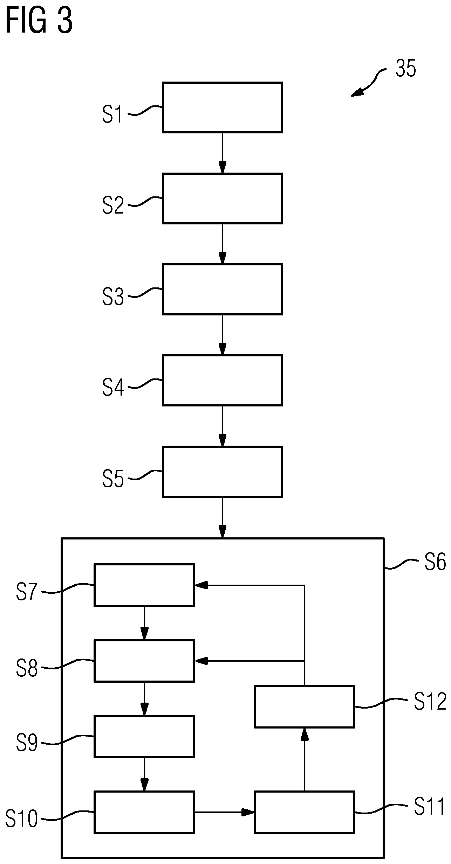

FIG 3 zeigt einen beispielhaften schematischen Ablaufplan 35 für ein Verfahren zum Herstellen des Pfadplanungsmoduls 24 und zum Betreiben des Robotersystems 1. In einem Verfahrensschritt S1 werden zunächst, insbesondere annotierte, Trainingsdaten für die KI-Komponente 25 erzeugt oder erfasst. Diese Trainingsdaten können etwa Beispielbewegungen des Roboters 11, zugehörige Kollisionsdaten und zugehörige Zielortvorgaben sowie eine Trainingszielvorgabe umfassen. Vorliegend werden als Trainingsdaten also als Grundlage zum Trainieren der KI-Komponente 25 für eine Pfadplanung für den Roboter 11, beispielsweise eine Vielzahl von Sensordaten von Positions- und Bewegungssensoren des Roboters 11 beziehungsweise des Roboterarms 14 sowie Sensordaten zu jeweils bevorstehenden oder erfolgten Kollisionen und Benutzerangaben oder Benutzervorgaben, welche die jeweilige Bewegung des Roboters 11 veranlasst haben, bereitgestellt.

In einem Verfahrensschritt S2 wird die KI-Komponente 25 mit den bereitgestellten Trainingsdaten vortrainiert. Ziel dieses Trainings ist es, die zu einer Kollision oder einer Gefahrensituation führenden Positionen oder Bewegungen des Roboters 11 von sicheren, also kollisionsfreien Positionen oder Bewegungen zu unterscheiden. Parameter können dabei beispielsweise jeweilige Posen, also Positionen und Orientierungen, einzelner Komponenten des Roboters 11, beispielsweise des hinteren Gelenks 16, der Strahlquelle 18 und des Detektors 20 sowie jeweilige Abstände des Roboters 11 zu umgebenden Fremdobjekten sein. Die KI-Komponente 25 wird dabei darauf trainiert, als die Ausgangsdaten 34 oder als Teil der Ausgangsdaten 34 einen sicheren, also kollisionsfreien Pfad für den Roboter 11 zu bestimmen, welchem der Roboter 11 kollisionsfrei von einer jeweiligen Ausgangspose hin zu dem jeweiligen Bewegungsziel oder Zielort folgen kann.

In einem Verfahrensschritt S3 wird nach Abschluss des Vortrainings der KI-Komponente 25 diese zusammen mit entsprechenden Schnittstellen zur Kommunikation, beispielsweise mit dem Steuergerät 10 für den Roboter 11, als das Pfadplanungsmodul 24 bereitgestellt.

Das Pfadplanungsmodul 24 kann dann beispielsweise im Rahmen einer Herstellung des Robotersystems 1 in dieses integriert werden.

In einem Verfahrensschritt S4 wird das Robotersystem 1 einschließlich des vortrainierten Pfadplanungsmoduls 24 an einem Betriebs- oder Einsatzort des Roboters 1 aufgebaut beziehungsweise installiert. Da jeder Einsatzort individuelle Gegebenheiten und Bedingungen aufweisen und dementsprechend individuelle Anforderungen oder Beschränkungen für den Betrieb des Roboters 11, insbesondere für Bewegungen des Roboterarms 14, bieten kann, erfolgt anschließend in einem Verfahrensschritt S5 ein Feintuning oder Nachtrainieren der KI-Komponente 25. Dabei wird die KI-Komponente 25 mit ortsspezifischen Trainingsdaten nachtrainiert, also an individuelle Gegebenheiten des jeweiligen Einsatzortes des Roboters 11 angepasst.

Ortsspezifische, also örtlich unterschiedliche, Bedingungen, an welche die KI-Komponente 25 dabei angepasst wird, können beispielsweise unterschiedliche Raumabmessungen und Raumformen, unterschiedliche Positionen von weiteren Geräten, wie beispielsweise dem Patiententisch 21, Anästhesieeinheiten und/oder dergleichen mehr relativ zu dem Roboter 11 sowie bevorzugte Standorte und Bewegungspfade oder Bewegungskorridore von Personal, welches bei einem Betrieb des Roboters 11 in dessen Umgebung oder Arbeitsbereich anwesend ist oder anwesend sein kann, umfassen.

Für das Feintuning oder Nachtrainieren der KI-Komponente 25, hier also insbesondere des DLNN, werden dabei bevorzugt wenigstens 10, besonders bevorzugt wenigstens 20, unterschiedliche Bewegungen des Roboters 11 durchgeführt und als Trainingsdaten der KI-Komponente 25 bereitgestellt. Dabei werden bevorzugt solche Bewegungen des Roboters 11 gewählt, welche typischen, an dem jeweiligen Einsatzort des Roboters 11 zu erwartenden Bewegungen oder Arbeitsabläufen entsprechen. Im vorliegenden Beispiel einer medizinischen oder medizintechnischen Anwendung können beispielsweise Bewegungen während vorgegebener Positionsübergänge eines Patienten während an dem jeweiligen Einsatzort geplanter Untersuchungen oder Behandlungen trainiert werden, beispielsweise ein Positionsübergang von einer lateralen Stellung zu einer AP-Stellung (anteriorposterior).

Ziel ist auch hier eine Unterscheidung zwischen unsicheren "Kollisionspositionen" und sicheren, kollisionsfreien Positionen des Roboters 11 und entsprechende Bewegungen oder Pfade zu trainieren. Dadurch kann die KI-Komponente 25 also lernen, wie, also entlang welcher Pfade oder mittels welcher Bewegungen oder Teilbewegungen, der Roboter 11 aus unterschiedlichen Ausgangspositionen heraus unterschiedliche Ziele sicher, also kollisionsfrei erreichen kann und welche Positionen oder Bewegungen zu Kollisionen führen können und daher zu vermeiden sind. Besonders vorteilhaft kann dies spezifisch für unterschiedliche Arbeitsabläufe, also beispielsweise für unterschiedliche Untersuchungen, Behandlungen oder Therapien, gelernt werden, bei denen unterschiedliche Gegebenheiten, beispielsweise hinsichtlich Anwesenheit und Positionen von Personal und Geräten, vorliegend können.

Nach Abschluss des Feintunings oder Nachtrainierens der KI-Komponente 25 ist das Robotersystem 1 an dem jeweiligen Einsatzort einsatzbereit für einen produktiven Einsatz. Im vorliegenden Beispiel kann dann also ein normaler oder regulärer Patientenbetrieb aufgenommen werden. Ein solcher Betrieb des Robotersystems 1 ist hier schematisch als Verfahrensschritt S6 angedeutet. Darin werden in einem Verfahrensschritt S7 zunächst die Eingangsdaten 29 erfasst. Die Eingangsdaten 29 können dabei beispielsweise Roboterdaten 30, Umgebungsdaten 31, Kollisionsdaten 32 und Benutzereingaben 33 umfassen.

Die Roboterdaten 30 können eine aktuelle Geometrie oder Pose des Roboters 11, situationsspezifische Details beispielsweise hinsichtlich eines aktuell an dem Roboterarm 14 befestigten oder gehaltenen Werkzeugs oder Geräts und/oder dergleichen mehr beschreiben.

Die Umgebungsdaten 31 können beispielsweise Daten sein, welche eine aktuelle räumliche oder geometrische Situation im Arbeitsbereich des Roboters 11 beschreiben. Vorliegend können die Umgebungsdaten 31 beispielsweise eine eingestellte Höhe, Ausrichtung, Verkippung, Bewegung, Geschwindigkeit, Beschleunigung und dergleichen des Patiententisches 21 angeben. Die Umgebungsdaten 31 können beispielsweise durch eine Sensorik jeweiliger Geräte oder des Roboters 11 selbst und/oder durch eine mit dem Robotersystem 1 verbundene Innenraumüberwachungseinrichtung, beispielsweise eine auf den Arbeitsbereich des Roboters 11 gerichtete Stereokamera oder dergleichen, bereitgestellt werden. Ebenso kann jeweiliges Personal beispielsweise über die Benutzerschnittstelle 4 Umgebungsdaten 31 vorgeben. Dies kann beispielsweise vorteilhaft sein, wenn bei einem bestimmten Einsatz des Roboters 11 eine einmalige oder außergewöhnliche Situation vorliegt, welche signifikant von trainierten Situationen oder Szenarien abweicht, beispielsweise durch Anwesenheit zusätzlichen Personals oder zusätzlicher Geräte, die nicht zum typischen Arbeitsablauf oder zur typischen Ausstattung an dem Einsatzort des Roboters 11 gehören.

Die Kollisionsdaten 32 können von einer Kollisions-, Annäherungs- oder Bewegungssensorik, beispielsweise des Roboters 11, bereitgestellt werden. Die Kollisionsdaten 32 können beispielsweise angeben, ob sich der Roboter 11 in Kontakt mit einem Fremdobjekt oder in unmittelbarer Nähe zu einem Fremdobjekt befindet.

Die Benutzereingaben 33 können wie beschrieben beispielsweise ein auszuführendes Untersuchungsprogramm oder eine Zielortvorgaben für den Roboter 11 umfassen.

In einem Verfahrensschritt S8 verarbeitet die KI-Komponente 25 die Eingangsdaten 29 und erzeugt dabei in Abhängigkeit von den Eingangsdaten 29 einen sicheren Pfad für den Roboter 11. Dieser sichere Pfad kann beispielsweise als Trajektorie, in Form von Wegpunkten oder als Abfolge von Posen oder dergleichen erzeugt werden, jeweils beispielsweise in einem vorgegebenen internen Koordinatensystem des Roboters 11 oder in einem Raumkoordinatensystem eines Raumes, in dem sich der Roboter 11 befindet, also etwa in einem weltfesten Koordinatensystem. Der durch die KI-Komponente 25 bestimmte oder geplante Pfad wird dann als Teil der Ausgangsdaten 34 an das Steuergerät 10 übermittelt.

Der durch die KI-Komponente 25 bestimmte Pfad kann dabei die Bewegung des Roboters 11 beispielsweise in wenigstens drei, bevorzugt wenigstens 6, besonders bevorzugt allen, Bewegungsfreiheitsgraden des Roboters 11 definieren. Um einen Berechnungs- oder Kostenaufwand zu begrenzen kann beispielsweise eine Beschränkung auf 20 Freiheitsgrade vorgesehen sein, welche mit heutzutage verfügbarer Berechnungshardware mit vertretbarem Aufwand realisierbar ist.

In einem Verfahrensschritt S9 empfängt das Steuergerät 10 die von der KI-Komponente 25 beziehungsweise dem Pfadplanungsmodul 24 ausgegebenen Ausgangsdaten 34 und setzt diese in Steueranweisungen für den Roboter 11 beziehungsweise für dessen Antrieb 13 um. Dazu kann das Steuergerät 10 beispielsweise eine aus dem Bereich der Robotik an sich bekannte Roboterkinematik oder Robotermodellierung anwenden.

In einem Verfahrensschritt S10 steuert das Steuergerät 10 den Roboter 11 gemäß den erzeugten Steueranweisungen, um den Roboter 11 beziehungsweise dem Roboterarm 14 entlang des von dem Pfadplanungsmodul 24 bereitgestellten Pfades zu bewegen.

Während und/oder nach dieser Bewegung des Roboters 11 wird in einem Verfahrensschritt S11 zusätzlicher Input erfasst und dem Pfadplanungsmodul 24 bereitgestellt. Dieser zusätzliche Input kann beispielsweise während der Bewegung aufgetretene Kollisionen oder potentiell kollisionsgefährliche Annäherungen des Roboters 11 an Fremdobjekte, beispielsweise bis auf weniger als einen vorgegebenen Abstandsschwellenwert, angeben oder beschreiben. Ebenso kann dieser zusätzliche Input beispielsweise während der Bewegung des Roboters 11 durch jeweiliges Personal vorgenommene manuelle Steuereingriffe, welche die Bewegung des Roboters 11 beeinflussen, umfassen oder beschreiben. Ebenso kann der zusätzliche Input beispielsweise Benutzereingaben des jeweiligen Personals umfassen, welche Vorlieben oder Anpassungen für bevorzugte Varianten oder Abweichungen von dem Pfad angeben.

Insbesondere kann der zusätzliche Input Bewegungsdaten umfassen, welche die tatsächlich von dem Roboter 11 ausgeführte Bewegung beschreiben und durch während oder nach dieser Bewegung erzeugte Annotationen ergänzt sind. Diese Annotationen können Kollisionen oder unsichere, also als unsicher eingestufte oder aus sonstigen Gründen zu vermeidende, Positionen oder Bewegungen des Roboters 11 angeben. Derartige Annotierungen können auf Basis der Kollisionsdaten 32 automatisch erzeugt werden, etwa jeweils bei Unterschreiten des Abstandsschwellenwertes. Ebenso können die Annotationen beispielsweise in Form von Benutzereingaben über die Benutzerschnittstelle 4 manuell erzeugt werden.

In einem Verfahrensschritt S12 wird die KI-Komponente 25 anhand des zusätzlichen Inputs nachtrainiert. Hier kann die KI-Komponente 25 also während das Robotersystem 1 sich bereits im produktiven Einsatz befindet, weiter optimiert und an den jeweiligen Einsatzort und Einsatzzweck angepasst werden.

Es ist beispielsweise möglich, dass nicht nur vollständige Bewegungen von einer Ausgangspose des Roboters 11 zu einem vorgegebenen Bewegungsziel nachtrainiert werden, sondern einzelne Teilbewegungen oder Bewegungsabschnitte dieser Bewegung einzeln nachtrainiert werden können. Führt der Roboter 11 beispielsweise eine Bewegung aus, welche zu einer kollisionsgefährlichen Situation führt oder aus sonstigen Gründen, beispielsweise gemäß einer Vorliebe des jeweiligen Personals, zu vermeiden ist, so kann der Roboter 11 beziehungsweise das Robotersystem 1 in einen handgeführten, gegebenenfalls kraftunterstützten, Betrieb versetzt werden. In diesem handgeführten Betrieb kann der Roboter 11 beziehungsweise der Roboterarm 14 in einer sicheren, kollisionsfreien Bewegung oder Teilbewegung entlang eines alternativen sicheren Pfades geführt werden. Die dabei ausgeführte Bewegung kann dann durch die KI-Komponente 25 als sichere Bewegung oder als sicherer Pfad in der jeweiligen Situation gelernt werden. Dieses Abfahren sicherer Bewegungen oder Pfade in einem jeweiligen Umfeld durch händisches Führen des Roboters 11 hat dabei den Vorteil, dass eindeutig sichere und von dem jeweiligen Personal bevorzugte Bewegungen oder Pfade durch die KI-Komponente 25 gelernt werden können, ohne dass diese Bewegungen oder Pfade manuell programmiert werden müssten. Damit ist dieses Nachtrainieren der KI-Komponente 25 vorteilhaft in einem Livebetrieb, also "on-the-fly" und damit besonders schnell, einfach und effizient möglich und von dem jeweils anwesenden Personal durchführbar.

Wie hier durch von dem Verfahrensschritt S12 zu den Verfahrensschritten S7 beziehungsweise S8 zurückführende Pfeile angedeutet, kann der Einsatz oder Betrieb des Roboters 11 beziehungsweise des Robotersystems 1 auf diese Weise kontinuierlich fortgeführt werden. Die hier beschriebene Betriebsschleife des Robotersystems 1 aus Pfadplanung, Bewegen des Roboters und Nachtrainieren der KI-Komponente 25 beziehungsweise des Pfadplanungsmoduls 24 kann also iterativ durchlaufen werden, sodass sich das Robotersystem 1 vorteilhaft kontinuierlich hinsichtlich einer Sicherheit und Effizienz optimieren und flexibel an neue Situationen oder Gegebenheiten an einem Einsatzort des Roboters 11 oder an neue Einsatzorte anpassen kann.

Insgesamt zeigen die beschriebenen Beispiele wie ein KI-Pfadplaner für robotische Systeme zum Verbessern einer Sicherheit und Effizienz bei deren Einsatz realisiert und verwendet werden kann.

## Patentansprüche

1. Verfahren (35) zum Herstellen eines Pfadplanungsmoduls (24) für einen Roboter (1, 11) zum Planen einer automatisch gesteuerten motorischen Bewegung des Roboters (1, 11), mit den Verfahrensschritten
- Bereitstellen einer trainierbaren KI-Komponente (25),
- Trainieren der KI-Komponente (25) für das Planen der Bewegung, indem der KI-Komponente (25) vorgegebene Eingangsdaten (29) und eine Trainingszielvorgabe für eine Bewertung der Eingangsdaten (29) zugeführt werden, wobei die Eingangsdaten (29) Bewegungsdaten (30), welche Beispielbewegungen des Roboters (1, 11) beschreiben, zugehörige Kollisionsdaten (32), welche bevorstehende und/oder erfolgte Kollisionen bei den Beispielbewegungen angeben, und zugehörige Zielortvorgaben (33) für die Beispielbewegungen umfassen und wobei die Trainingszielvorgabe umfasst, unsichere Bewegungen, die zu einer Kollision des Roboters (1, 11) mit einem Fremdobjekt führen können, von sicheren, kollisionsfreien Bewegungen zu unterscheiden und einen jeweiligen Pfad von einer jeweiligen Ausgangspose des Roboters (1, 11) zu dem jeweiligen Zielort zu finden, dem der Roboter (1, 11) unter Ausführung lediglich sicherer Bewegungen folgen kann, und
- Bereitstellen der trainierten KI-Komponente (25) mit zur Kommunikation mit dem Roboter (1, 11) eingerichteten Eingangs- und Ausgangsschnittstellen als das Pfadplanungsmodul (24) .

2. Verfahren (35) nach Anspruch 1, **dadurch gekennzeichnet, dass** die Bewegungsdaten (30) von dem Roboter (1, 11) während der Beispielbewegung eingenommene Posen aufgelöst nach einzeln beweglichen Segmenten (15, 17) des Roboters (1, 11) angeben.

3. Verfahren (35) nach einem der vorhergehenden Ansprüche, **dadurch gekennzeichnet, dass** das Trainieren der KI-Komponente (25) ein Vortrainieren und ein späteres Feinabstimmen anhand von ortsspezifischen Eingangsdaten (29), die spezifisch sind für einen individuellen Einsatzort des Roboters (1, 11), umfasst.

4. Verfahren (35) nach Anspruch 3, **dadurch gekennzeichnet, dass** die ortsspezifischen Eingangsdaten (29) räumliche Bedingungen (31) und für den individuellen Roboter (1, 11) an dessen individuellem Einsatzort vorgesehene Arbeitsabläufe beschreiben.

5. Verfahren (35) nach einem der vorhergehenden Ansprüche, **dadurch gekennzeichnet, dass** das Trainieren der KI-Komponente (25) während eines produktiven Einsatzes des Roboters (1, 11) anhand von Bewegungsdaten (30) dieses Roboters (1, 11) fortgesetzt wird.

6. Verfahren (35) nach Anspruch 5, **dadurch gekennzeichnet, dass** für das fortgesetzte Training der KI-Komponente (25) Bewegungsdaten (30), welche tatsächlich von dem Roboter (1, 11) ausgeführte Bewegungen beschreiben, die zu einer Kollision oder einer Kollisionsgefahr geführt haben, dem Pfadplanungsmodul (24) automatisch als unsicher gekennzeichnete Trainingsdaten zugeführt werden.

7. Verfahren (35) nach einem der vorhergehenden Ansprüche, **dadurch gekennzeichnet, dass** für das Trainieren der Roboter (1, 11) in einem handgeführten Betrieb entlang wenigstens einer kollisionsfreien Trajektorie geführt wird, diese Trajektorie aufgezeichnet und als sicher gekennzeichnet und dann der KI-Komponente (25) zugeführt wird.

8. Verfahren (35) zum Betreiben eines motorisch bewegbaren Roboters (1, 11), bei dem
- einem gemäß einem der vorhergehenden Ansprüche hergestellten Pfadplanungsmodul (24) eine aktuelle Ausgangspose des Roboters (1, 11) und ein Bewegungsziel, welches durch die Bewegung erreicht werden soll, vorgegeben werden,
- durch des Pfadplanungsmodul (24) automatisch ein Pfad bestimmt, entlang welchem der Roboter (1, 11) von der Ausgangspose zum Erreichen des Bewegungsziels durch kollisionsfreie Bewegungen folgen kann,
- der bestimmte Pfad einem Steuergerät (10) des Roboters (1, 11) bereitgestellt und durch das Steuergerät (10) automatisch in Steueranweisungen für einen Antrieb (13) des Roboters (1, 11) umgesetzt wird, und
- der Antrieb durch das Steuergerät (10) automatisch gemäß den Steueranweisungen angesteuert wird, um den Roboter (1, 11) entlang des bestimmten Pfades zu bewegen.

9. Pfadplanungsmodul (24) für einen motorisch bewegbaren Roboter (1, 11) zum Planen einer automatisch gesteuerten motorischen Bewegung des Roboters (1, 11), wobei das Pfadplanungsmodul (24) gemäß einem Verfahren (35) nach einem der Ansprüche 1 bis 7 hergestellt und/oder zum Betreiben gemäß einem Verfahren (35) nach Anspruch 8 eingerichtet ist.

10. Roboter (1, 11), aufweisend einen motorisch bewegbaren Roboterarm (14), einen Antrieb (13) zum Bewegen des Roboterarms (14) und ein mit dem Antrieb (13) verbundenes Steuergerät (10) zum automatischen Ansteuern des Antriebs (13), wobei der Roboter (1, 11) ein Pfadplanungsmodul (24) nach Anspruch 9 aufweist.
